# EUROPEAN PATENT SPECIFICATION

(11) **EP 2 276 848 B1**
(45) Date of publication and mention of the grant of the patent: **17.12.2014**
(21) Application number: 09739766.5
(22) Date of filing: 30.04.2009
(51) Int. Cl.: C12P 19/02, C12P 7/06, C12P 19/18

(54) **ENHANCED FERMENTATION PROCESS USING MOLASSES**
INTENSIVIERTER GÄRPROZESS MIT MOLASSEN
PROCÉDÉ DE FERMENTATION AMÉLIORÉ UTILISANT DES MÉLASSES

(30) Priority: 30.04.2008 US 49363 P
(43) Date of publication of application: 26.01.2011
(73) Proprietor: Danisco US Inc., Palo Alto, CA 94304 (US)
(72) Inventor: DUAN, Gang, Palo Alto, CA 94304 (US); RUAN, Zhenhua, Palo Alto, CA 94304 (US); SHETTY, Jayarama, K., Palo Alto, CA 94304 (US); XU, Hongxian, Palo Alto, CA 94304 (US)
(74) Representative: Kiddle, Simon John
(86) International application number: PCT/US2009/042224
(87) International publication number: WO 2009/134964

(56) References cited:
- WO-A1-96/13600
- WO-A2-2005/100583
- US-A- 3 836 432
- US-A- 4 855 232
- ANONYMOUS: "Modification of beer character using transglucosidase" ENZYME WAVE, [Online] 14 January 2006 (2006-01-14), page 6, XP002564584 Retrieved from the Internet: URL:http://web.archive.org/web/20060114134 107/http://www.amano-enzyme.co.jp/jp/produ ctuse/pdf/beer.pdf> [retrieved on 2010-01-21]
- BILFORD ET AL.: "Alcoholic fermentation of molasses" INDUSTRIAL AND ENGINEERING CHEMISTRY, vol. 34, no. 11, 1942, XP002565959
- SCIGELOVA MICHAELA ET AL: "Purification of alpha-galactosidase from Aspergillus niger for application in the synthesis of complex oligosaccharides" JOURNAL OF MOLECULAR CATALYSIS B ENZYMATIC, vol. 8, no. 4-6, 18 February 2000 (2000-02-18), pages 175-181, XP002564586 ISSN: 1381-1177
- SCIGELOVA MICHAELA ET AL: "Glycosidases-A great synthetic tool" JOURNAL OF MOLECULAR CATALYSIS B ENZYMATIC, vol. 6, no. 5, 5 May 1999 (1999-05-05), pages 483-494, XP002564587 ISSN: 1381-1177
- DATABASE WPI Week 197729 Thomson Scientific, London, GB; AN 1977-51663Y XP002565880 & SU 539 069 A (FERMENTATION PROD) 29 December 1977 (1977-12-29)

## Description

### FIELD OF THE INVENTION

The present invention relates to methods of utilizing at least one transglucosidase enzyme to increase the amount of fermentable sugars in molasses fermentation processes. The transglucosidase enzyme can be used alone or in combination with other carbohydrate processing enzymes.

### BACKGROUND OF THE INVENTION

Molasses typically refers to a by-product from sugarcane and beet processing. Molasses is produced globally in very large amounts. For instance, in the year 2005, molasses production globally was estimated at 50.7 million tons. About 48% of the total molasses was produced in Asia, and the major share of that was produced in India, China and Thailand. The molasses produced from cane and beets each has a similar sugar composition. Both types of molasses contain both fermentable and non-fermentable sugars. However, beet molasses contains a lower concentration of fermentable sugars and a higher concentration of non-fermentable sugars than cane molasses. Industrial fermentations predominately use glucose and sucrose as feedstock for the production of a multitude of proteins, enzymes, amino acids, alcohols, organic acids, pharmaceuticals and other biochemicals. However, in many applications, molasses can also be used in fermentations.

Typically, the total composition of molasses from sugarcane or beet sugar (sugars, proteins, etc) contains significant amounts of proteins, non-fermentable starch and non-fermentable oligosaccharides such as raffinose, a tri-saccharide (galactosyl- glucosyl -fructose), and stachyose, a tetra -saccharide (galactosyl-galactosyl - glucosyl- fructose). These non-fermentable sugars cannot be used in the fermentation process because the enzymes used in previous processes have not hydrolyzed raffinose and stachyose to fermentable sugars. While α-Galactosidase, was reported to be capable of hydrolyzing non-fermentable sugars (See e.g., Suzuki et.al. USP 3,767,526, 1973; and Meguro et al, USP, 4,036,694, 1977), it did not hydrolyze raffinose and stachyose. Dextranase was also used to hydrolyze dextrins in sugar solutions (Murtaugh, J.E. 1999 Molasses as a feedstock for alcohol production. In: The Alcohol Textbook, 3rd Ed. K.A. Jacques, T.P. Lyons and D.R. Kelsall, eds Nottingham University Press, UK) but only worked on short-chain dextrins and did not hydrolyze non-fermentable sugars at all. Thus, better methods for enhancing the fermentability of molasses are needed.

International patent application WO96/13600 discloses fermentation processes using molasses but the use of fungal transglucosidases is not disclosed in this document.

### SUMMARY OF THE INVENTION

The invention in its broadest form is defined in independent claims 1 and 15:
1. A method for increasing the fermentation yield of molasses in a fermentation medium, wherein the molasses contains at least one of the non-fermentable sugars raffinose and/or stachyose, the method comprising contacting the molasses with an enzyme composition comprising at least one fungal transglucosidase capable of hydrolyzing non-fermentable sugars such as raffinose and/or stachyose.
15. A method of producing ethanol from molasses which comprises having treated molasses in a fermentation medium according to the method of any one of the preceding claims, the further step of fermenting the molasses in the presence of a fermenting organism to produce ethanol.

Preferred embodiments are defined in the dependent claims 2-14:
2. The method of claim 1, wherein the molasses is blackstrap molasses from sugarcane* high test cane molasses, refiners cane or beet molasses produced as a byproduct of refining raw brown sugar to produce white sugar, beet molasses from sugar beets, and citrus molasses juices extracted from dried citrus pulp.
3. The method of claim 1 or claim 2, further comprising the addition of one or more secondary enzymes to hydrolyze granular starch, proteins and/or residual starch in the molasses.
4. The method of any one of the preceding claims, wherein fermentable sugars from molasses in the fermentation reaction are converted to obtain end products, such as an alcohol, an organic acid or a specialty biochemical.
5. The method of claim 4, wherein the alcohol is ethanol and/or the organic acid is lactic acid or citric acid and/or the specialty biochemical is an amino acid.
6. The method of any one of the preceding claims, wherein the method increases the fermentable sugars obtained from the molasses.
7. The method of claim 6, further comprising fermenting the fermentable sugars to end products in the presence of fermenting microorganisms.
8. The method of claim 7, wherein the contacting and fermenting occur simultaneously or wherein the contacting is a pretreatment.
9. The method of claim 7, wherein the molasses comprises non-fermentable starch and the contacting, pretreatment and/or fermenting occurs at a temperature below the gelatinization temperature of the granular starch.
10. The method of claim 9, wherein the non-fermentable starch is granular starch.
11. The method of claim 9, wherein:
   (a) the contacting and/or pretreatment temperature is below 52°C; and/or
   (b) the fermentation temperature is from 15 to 40°C; and/or
   (c) the fermentation, contacting and pretreatment temperature is from 15 to 40° C; and/or
   (d) the contacting and/or pretreatment is conducted at a pH from 2.0 to 7.0; and/or
   (e) the fermentation is conducted at a pH from 3.5 to 7.0.
12. The method of claim 6, further comprising contacting the molasses with at least one granular starch hydrolyzing enzyme (GSHE), such as an alpha amylase or a glucoamylase.
13. The method of claim 6, further comprising contacting the molasses with at least one other enzyme, such as a hemicellulase, a cellulase, a pectinase, a protease, Pglucosidase, a non-GSHE alpha amylase, and/or a non-GSHE glucoamylase.
14. The method of claim 13, wherein the protease is an acid fungal protease, such as an acid fungal protease is from an Aspergillus or Trichoderma species.

### BRIEF DESCRIPTION OF THE DRAWINGS

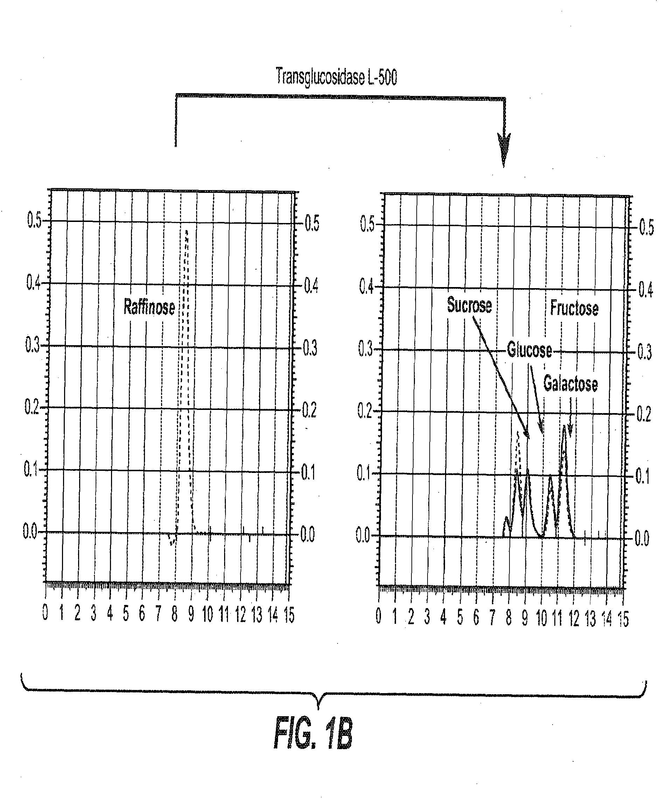

Figure 1, panel A is a diagram of the chemical reaction that takes place to produce the chromatogram in Figure 1, panel B. Figure 1, panel B is an HPLC chromatogram of raffinose incubated with TRANSGLUCOSIDASE L-500, pH 4.5, 60°C.

### DETAILED DESCRIPTION OF THE INVENTION

The invention is based on the finding that fungal transglucosidase hydrolyzes non-fermentable sugars into fermentable sugars. This was identified when a variety of enzymes were tested on molasses for the ability to increase the alcohol yield in molasses fermentations. While some enzymes that were tested increased the alcohol yield moderately, the addition of TRANSGLUCOSIDASE L-500 (a commercial product from Danisco US, Inc., Genencor division) to the fermentation, resulted in a significant increase in the yield of alcohol. Further tests herein showed that the transglucosidase was able to hydrolyze non-fermentable oligosaccharides such as raffinose and stachyose into fermentable sugars. This was unexpected since transglucosidases are generally known for converting malto-oligosaccharides into isomalto-oligosaccharides such as isomaltose and panose which are less fermentable. Thus, it was not expected that the addition of a transglucosidase to molasses would result in hydrolysis of raffinose and stachyose (tri-saccharides) nor was it expected that they would be hydrolyzed to fermentable mono-saccharides.

The invention provides novel processes for increasing the fermentation yield of molasses using a transglucosidase during or prior to fermentation. Some embodiments of the process include methods of contacting molasses with an enzyme composition or blend including at least one transglucosidase. In some embodiments, this results in the hydrolysis of non-fermentable sugars like raffinose and stachyose into fermentable sugars. In some embodiments, the contacting occurs at a temperature below the starch gelatinization temperature of the granular starch in the molasses. The method can also include the addition of secondary enzymes to hydrolyze, for example, granular starch, proteins and residual starch in molasses. The invention also relates to the conversion of the fermentable sugars from molasses to obtain end products, such as alcohol (e.g., ethanol), organic acids (lactic acid, citric acid) and specialty biochemical (amino acids, monosodium glutamate, etc).

As shown in the examples herein, the initial finding transglucosidase increased the yield of ethanol in molasses fermentation can be explained by the hydrolysis of raffinose and stachyose (two non-fermentable sugars in the molasses) into fermentable sugars. Further, other starch hydrolyzing enzymes, such as raw starch hydrolyzing enzymes to the yeast fermentation of molasses also increased the amount of fermentable sugars in the process. For example, the addition of alpha amylase and glucoamylase with granular starch hydrolyzing properties (GSHE) also produced more fermentable sugars. Secondary enzymes, such as proteases also improved the fermentation. For example, Acid fungal protease (FERMGEN ™, GC 106 from Danisco US, Inc., Genencor Division), was found to effectively improve fermentation by hydrolyzing the protein in molasses, producing free amino nitrogen (FAN) and functional peptides which increased the availability of nitrogen and enhanced the yeast growth. Enzymes such as cellulases, hemicellulases, beta-glucosidases, beta-glucanases, dextranases and pectinases can also be used in the hydrolysis of non-starch-polysaccharides and gum in molasses. The present invention provides methods and compositions for improving the yield and efficiency of molasses fermentations to ethanol.

In some embodiments, the method involves incubating and/or fermenting molasses at a temperature conducive to fermentation by a fermentation organism (e.g., 28-38°C) in the presence of transglucosidase, resulting in a higher fermentation yield than a fermentation without the tranglucosidase. In some embodiments, the temperature is below the starch gelatinization temperature of granular starch in the molasses. In other embodiments secondary enzymes can be included, such as GSHE's, proteases, pectinases, beta-glucanases, xylanases, and cellulases with the at least one transglucosidase. In some embodiments, the transglucosidase and optionally at least one secondary enzyme is added in a pretreatment step and then the fermentation is conducted.

### Definitions

Unless otherwise indicated, the practice of the invention involves conventional techniques commonly used in molecular biology, protein engineering, recombinant DNA techniques, microbiology, cell biology, cell culture, transgenic biology, immunology, and protein purification, which are within the skill of the art. Such techniques are known to those of skill in the art and are described in numerous texts and reference works.

Unless defined otherwise herein, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described. Accordingly the terms defined immediately below are more fully described by reference to the Specification as a whole. Also, as used herein, the singular "a", "an" and "the" includes the plural reference unless the context clearly indicates otherwise. Numeric ranges are inclusive of the numbers defining the range. Thus, for example, reference to a composition containing "a compound" includes a mixture of two or more compounds. It should also be noted that the term "or" is generally employed in its sense including "and/or" unless the content clearly dictates otherwise. Unless otherwise indicated amino acids are written left to right in amino to carboxy orientation, respectively.

As used herein, "Molasses" refers to a syrup produced as a by-product of the processing of sugarcane or other plant products. There are many types of molasses, including for example, blackstrap molasses, a by-product of sugar production from sugarcane; high test (cane) molasses, a primary product extracted from sugarcane; refiners cane molasses, a by-product of refining raw brown sugar to produce white sugar; beet molasses, a by-product of sugar production from sugar beets; and citrus molasses: juices extracted from the manufacture of dried citrus pulp, to name a few.

As used herein, "Fermentable sugars" are sugars that can be directly digested by fermentation organisms (e.g. yeast). Some examples of fermentable sugars include fructose, maltose, glucose, sucrose, and galactose.

As used herein "Non-fermentable sugars" are sugars that can not be directly digested by fermentation organisms (e.g. yeast). Some examples of non-fermentable sugars include raffinose and stachyose.

As used herein "Alpha amylases" are α -1,4-glucan-4-glucanohydrolases (E.C. 3.2.1.1) and have the ability to cleave or hydrolyze internal α -1,4 -glycosidic linkages in starch (*e.g.* amylopectin or amylose polymers).

"Dextrins" are used herein to refer to short chain polymers of glucose (e.g., 2 to 10 units).

As used herein the term "starch" refers to any material comprised of the complex polysaccharide carbohydrates of plants, comprised of amylose and amylopectin with the formula (C₆H₁₀O₅)ₓ, wherein x can be any number.

As used herein, the term "granular starch" means raw starch, that is, starch which has not been subject to temperatures of gelatinization.

As used herein, the terms "granular starch hydrolyzing (GSH) enzyme (GSHE)" and "enzymes having granular starch hydrolyzing (GSH) activity" refer to enzymes which have the ability to hydrolyze starch in granular form.

As used herein, the term "glucoamylase (e.g., E.C. 3.2.1.3)" refers to any enzyme that is capable of catalyzing the release of D-glucose from the non-reducing ends of starch and related oligo-and polysaccharides.

As used herein, the term "transglucosidase" refers to enzymes that are capable of performing glycosyl transfer reactions. Transglucosidases can be divided into α-1,6, α-1,4, α-1,3, α-1,2 glycosyl transferases. Some transglucosidases are D-glucosyltransferases (see e.g., E.C. 2.4.1) and catalyze both transfer and some hydrolytic reactions on incubation with α-D-gluco-malto-oligosaccharides. Transfer occurs most frequently to HO-6, producing isomaltose from D-glucose, and panose from maltose. The enzyme can also form kojibiose or nigerose or maltose. As a result of transglucosidase reactions, the malto-oligosaccharides are converted to isomalto-oligosaccharides resulting in a new class of polysaccharides containing high proportions of glucosyl residues linked by an α-D-1,6 linkage from the non-reducing end.

As used herein, the term "oligosaccharides" refers to any compound having 2 to 10 monosaccharide units joined in glycosidic linkages. These short chain polymers of simple sugars include dextrins.

As used herein, the term "DE" or "dextrose equivalent" is an industry standard for measuring the concentration of total reducing sugars, calculated as D-glucose on a dry weight basis. Unhydrolyzed granular starch has a DE that is essentially 0 and D-glucose has a DE of 100.

As used herein, the term "glucose syrup" refers to an aqueous composition containing glucose. Glucose syrup will typically have a DE of at least 20. In some embodiments, glucose syrup will not contain more than 21% water. In some embodiments, the glucose syrup will not contain less than 25% reducing sugar calculated as dextrose. In some embodiments, glucose syrup will include at least about 90% D-glucose and in another embodiment glucose syrup will include at least about 95% D-glucose. In some embodiments the terms syrup and glucose syrup are used interchangeably.

As used herein, the term "total sugar content" refers to the total amount of sugar present in a starch composition.

As used herein, the term "dry solids (ds)" refers to the total solids of a slurry in % on a dry weight basis.

As used herein, the term "gelatinization" means solubilization of a starch molecule, generally by cooking, to form a viscous suspension.

As used herein, the term "gelatinization temperature" refers to the temperature at which gelatinization of a starch-containing substrate begins. The exact temperature of gelatinization depends on the specific starch and may vary depending on factors such as plant species and environmental and growth conditions. The initial starch gelatinization temperature ranges for a number of granular starches, for example, include barley (52°C to 59°C), wheat (58°C to 64°C), rye (57°C to 70°C), corn (62°C to 72°C), high amylose corn (67°C to 80°C), rice (68°C to 77°C), sorghum (68°C to 77°C), potato (58°C to 68°C), tapioca (59°C to 69°C) and sweet potato (58°C to 72°C). (See, e.g., J.J.M. Swinkels pg 32 - 38 in STARCH CONVERSION TECHNOLOGY, Eds Van Beynum et al., (1985) Marcel Dekker Inc. New York and The Alcohol Textbook 3rd ED. A Reference for the Beverage, Fuel and Industrial Alcohol Industries, Eds Jacques et al., (1999) Nottingham University Press, UK).

As used herein, the term "below the gelatinization temperature" refers to a temperature that is less than the gelatinization temperature.

As used herein, the term "fermentation" refers to the enzymatic and anaerobic breakdown of organic substances by microorganisms to produce simpler organic compounds. While fermentation occurs under anaerobic conditions it is not intended that the term be solely limited to strict anaerobic conditions, as fermentation also occurs in the presence of oxygen.

As used herein, the term "end product" refers to any carbon-source derived product which is enzymatically converted from a fermentable substrate. In some embodiments, the end product is an alcohol (e.g., ethanol).

As used herein, the term "derived" encompasses the terms "originated from", "obtained" or "obtainable from", and "isolated from" and in some embodiments as used herein means that a polypeptide encoded by the nucleotide sequence is produced from a cell in which the nucleotide is naturally present or in which the nucleotide has been inserted.

As used herein the terms "fermenting organism" and "fermenting microorganism" refer to any microorganism or cell, which is suitable for use in fermentation for directly or indirectly producing an end product,

As used herein the term "ethanol producer" or ethanol producing microorganism" refers to a fermenting organism that is capable of producing ethanol from a mono- or oligosaccharide.

As used herein, the terms "recovered", "isolated", and "separated" as used herein refer to a protein, cell, nucleic acid or amino acid that is removed from at least one component with which it is naturally associated.

As used herein, the terms "protein" and "polypeptide" are used interchangeability herein. In the present disclosure and claims, the conventional one-letter and three-letter codes for amino acid residues are used. The 3-letter code for amino acids as defined in conformity with the IUPAC-IUB Joint Commission on Biochemical Nomenclature (JCBN). It is also understood that a polypeptide can be coded for by more than one nucleotide sequence due to the degeneracy of the genetic code.

As used herein, the term "contacting" refers to the placing of at least one enzyme in sufficiently close proximity to its respective substrate to enable the enzyme(s) to convert the substrate to at least one end product. In some embodiments, the end product is a "product of interest" (i.e., an end product that is the desired outcome of the fermentation reaction). Those skilled in the art will recognize that mixing at least one solution comprising the at least one enzyme with the respective enzyme substrate(s) results in "contacting."

Although any methods and materials similar or equivalent to those described herein can be used in the practice or testing of the present invention, exemplary and preferred methods and materials are now described.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

The publications discussed herein are provided solely for their disclosure prior to the filing date of the present application. Nothing herein is to be construed as an admission that the present invention is not entitled to antedate such publication by virtue of prior invention.

### Exemplary embodiments

The inventors have found addition of a transglucosidase to a molasses fermentation and/or as a pretreatment of the molasses provides advantages over the fermentation of molasses without addition of a transglucosidase. Further, addition of a granular starch hydrolyzing enzyme (GSHE) such as an alpha amylase (AA) and/or a glucoamylase (GA) provided further advantages. Addition of any of the following enzymes alone or in combination with the transglucosidase and optionally the GSHE, provided further advantages: a glucoamylase, a cellulase, a hemicellulase, a β-glucanase, a β-glucosidase, a pectinase, and/or an acid fungal protease. In some embodiments, at least one alpha amylase and/or at least one glucoamylase is added. In some embodiments, the at least one AA and/or GA has granular starch hydrolyzing (GSH) activity.

In one aspect, the present invention relates to an enzyme blend or composition comprising a transglucosidase in combination with at least one secondary enzyme chosen from: a glucoamylase, an alpha amylase, a granular starch hydrolyzing enzyme, a cellulase, a hemicellulase, a β-glucanase, a β-glucosidase, a pectinase, and an acid fungal protease. The invention also relates to the use of the blend or composition in the production of fermentable sugars in molasses and the production of end products (e.g., ethanol). In further aspects the invention relates to an enzyme blend or composition comprising a tranglucosidase and at least one GHSE. The GSHE can be an alpha amylase and/or a glucoamylase. In further embodiments, the invention relates to an enzyme blend or composition comprising a tranglucosidase, an alpha amylase with GHSE activity, and a glucoamylase with GSHE activity. In further embodiments, the combination of the transglucosidase, the AA, and the GA, further comprises at least one of the following secondary enzymes: an acid fungal protease (AFP), a cellulase, a hemi-cellulase, a β-glucanase, a β-glucosidase, and a pectinase. One advantage of the blend or composition comprising TG and optionally a GSHE is that it results in a greater amount of ethanol relative to the amount of ethanol produced by fermentation alone under substantially the same conditions. In some aspects, the increase is greater than 0.1%, relative to fermentation alone, including greater than 0.2%, 0.3%, 0.4%, 0.5%, 0.6%, 0.7%, 0.8%, 0.9%, 1.0%, 1.5%, 2%, and 2.5%.

### Transglucosidase

Any suitable fungal transglucosidase finds use in the methods of the invention. The type of transglucosidase is not critical to the invention. In some embodiments, the transglucosidase is an enzyme that is capable of performing glycosyl transfer reactions. In other embodiments, the transglucosidase is also able to hydrolyze non-fermentable sugars such as raffinose and stachyose to fermentable sugars. Typically transglucosidases (see e.g., E.C. 2.4.1) are enzymes that are capable of performing glycosyl transfer reactions. Transglucosidases can be divided into α-1,6, α-1,4, α-1,3, α-1,2 glycosyl transferases. Some transglucosidases useful in the invention are D-glucosyltransferases (see e.g., E.C. 2.4.1.25) and catalyze both transfer reactions and some hydrolytic reactions on incubation with α-D-gluco-oligosaccharides. Transfer occurs most frequently to HO-6, producing isomaltose from D-glucose, and panose from maltose. The enzyme can also form kojibiose or nigerose or maltose. As a result of transglucosidase reactions, malto-oligosaccharides are converted to isomalto-oligosaccharides resulting in a new class of polysaccharides containing high proportions of glucosyl residues linked by an α -D-1,6 linkage from the non-reducing end. The activity of transglucosidases is measured as Transglucosidase units (TGU). Fungal transglucosidases can be derived from the heterologous or endogenous protein expression of bacteria, plants and fungal sources. Some transglucosidases useful in the invention are produced by several strains of filamentous fungi and yeast, in particular, transglucosidases secreted from strains of *Aspergillus.* Transglucosidase enzymes that may be employed in the subject compositions are generally described in Barker et al (Studies of Aspergillus niger. Part II. Transglycosidation by Aspergillus niger. J. Chem. Soc. 1953 3588-3593); Pazur et al (The glycoprotein nature and antigenicity of a fungal D-glucosyltransferase. Carbohydr. Res. 1986 149:137-47) and Nakamura et al (Cloning and sequencing of an alpha-glucosidase gene from Aspergillus niger and its expression in A. nidulans J. Biotechnol. 1997 53:75-84). In particular embodiments, the transglucosidase enzyme that may be employed may be purchased from Megazyme (Co. Wicklow, Ireland) or Danisco US, Inc. Genencor Division (Palo Alto, CA under the trade name TRANSGLUCOSIDASE L-500). In some embodiments, the enzyme can be an *Aspergillus niger* transglucosidase enzyme produced in *Trichoderma reesei* cells. In certain cases, the transglucosidase enzyme may be a wild type fungal transglucosidase (e.g., a fungal transglucosidase having an amino acid sequence deposited in NCBI's Genbank database as accession numbers: D45356 (GID:2645159; *Aspergillus niger),* BAD06006.1 (GID:4031328; *Aspergillus awamori*), BAA08125.1 (GID:1054565; *Aspergillus oryzae*), XP_001210809.1 (GID:115492363; *Aspergillus terreus),* XP_001271891.1 (GID:121707620; *Aspergillus clavatus*), XP_001266999.1 (GID:119500484; *Neosartorya fischeri*), XP_751811.1 (GID:70993928; *Aspergillus fumigatus*), XP_659621.1 (GID:67523121; *Aspergillus nidulans*), XP_001216899.1 (GID:115433524; *Aspergillus terreus*) and XP_001258585.1 (GID:119473371; *Neosartorya fischeri*), or a variant thereof that has an amino acid sequence that is at least 70% identical, e.g., at least 80% identical, at least 85% identical, at least 90% identical, at least 95% identical, or at least 98% identical to a wild type fungal transglucosidase. It is not intended that the present invention be limited to any specific transglucosidase, as any suitable transglucosidase finds use in the methods of the present invention. Indeed, it is not intended that the present invention be limited to the specifically recited transglucosidases and commercial enzymes.

### Secondary enzymes:

### Glucoamylases

Various glucoamylases (GA) (E.C. 3.2.1.3.) find use in the present invention. In some embodiments, the glucoamylase having use in the invention has granular starch hydrolyzing activity (GSH) or is a variant that has been engineered to have GSH activity. In some embodiments, GSH activity is advantageous because the enzymes act to break down more of the starch in the molasses, particularly any granular starch. In some embodiments, the glucoamylases are endogenously expressed by bacteria, plants, and/or fungi, while in some alternative embodiments, the glucoamylases are heterologous to the host cells (e.g., bacteria, plants and/or fungi). In some embodiments, glucoamylases useful in the invention are produced by several strains of filamentous fungi and yeast. For example, the commercially available glucoamylases produced by strains of *Aspergillus* and *Trichoderma* find use in the present invention. Suitable glucoamylases include naturally occurring wild-type glucoamylases as well as variant and genetically engineered mutant glucoamylases (e.g. hybrid glucoamylases). Hybrid glucoamylase include, for example, glucoamylases having a catalytic domain from a GA from one organism (e.g., *Talaromyces* GA) and a starch binding domain (SBD) from a different organism (e.g.; *Trichoderma* GA). In some embodiments, the linker is included with the starch binding domain (SBD) or the catalytic domain. The following glucoamylases are nonlimiting examples of glucoamylases that find use in the processes encompassed by the invention. *Aspergillus niger* G1 and G2 glucoamylase (See e.g., Boel et al., (1984) EMBO J. 3:1097 - 1102; WO 92/00381, WO 00/04136 and USP 6,352,851); *Aspergillus awamori* glucoamylases (See e.g., WO 84/02921); *Aspergillus oryzae* glucoamylases (See e.g., Hata et al., (1991) Agric. Biol. Chem. 55:941 - 949) and *Aspergillus shirousami.* (See e.g., Chen et al., (1996) Prot. Eng. 9:499 - 505; Chen et al. (1995) Prot. Eng. 8:575-582; and Chen et al., (1994) Biochem J. 302:275-281).

Secondary glucoamylases that find use in the present invention also include those obtained from strains of *Talaromyces* ((e.g., *T. emersonii, T. leycettanus, T. duponti* and *T. thermophilus* glucoamylases (See e.g., WO 99/28488; USP No. RE: 32,153; USP No. 4,587,215)); strains of *Trichoderma,* (e.g., *T. reesei*) and glucoamylases having at least about 80%, about 85%, about 90% and about 95% sequence identity to SEQ ID NO: 4 disclosed in US Pat. Pub. No. 2006-0094080; strains of *Rhizopus,* (e.g., *R. niveus and R. oryzae*); strains of *Mucor* and strains of *Humicola,* ((e.g., *H. grisea* (See, e.g., Boel et al., (1984) EMBO J. 3:1097-1102; WO 92/00381; WO 00/04136; Chen et al., (1996) Prot. Eng. 9:499-505; Taylor et al., (1978) Carbohydrate Res. 61:301-308; USP. 4,514,496; USP 4,092,434; USP 4,618,579; Jensen et al., (1988) Can. J. Microbiol. 34:218 - 223 and SEQ ID NO: 3 of WO 2005/052148)). In some embodiments, the glucoamylase useful in the invention has at least about 85%, about 90%, about 92%, about 94%, about 95%, about 96%, about 97%, about 98% and about 99% sequence identity to the amino acid sequence of SEQ ID NO: 3 of WO 05/052148. Other glucoamylases useful in the present invention include those obtained from *Athelia rolfsii* and variants thereof (See e.g., WO 04/111218) and *Penicillium* spp. (e.g., *Penicillium chrysogenum*).

Commercially available glucoamylases useful in the invention include but are not limited to DISTILLASE®, OPTIDEX® L-400 and G ZYME® G990 4X, GC480, G-ZYME® 480, FERMGEN® 1-400 (Danisco US, Inc, Genencor Division), CU.CONC® (Shin Nihon Chemicals, Japan), GLUCZYME® (Amano Pharmaceuticals, Japan (See e.g. Takahashi et al., (1985) J. Biochem. 98:663-671)). Secondary enzymes that find use in the invention include three forms of glucoamylase (e.g., E.C.3.2.1.3) produced by a *Rhizopus sp.,* namely "Glucl" (MW 74,000), "Gluc2" (MW 58,600) and "Gluc3" (MW 61,400). It is not intended that the present invention be limited to any specific glucoamylase as any suitable glucoamylase finds use in the methods of the present invention. Indeed, it is not intended that the present invention be limited to the specifically recited glucoamylases and commercial enzymes.

### Alpha amylase

Various alpha amylases find use in the methods of the invention. In some embodiments, the alpha amylase having use in the invention has granular starch hydrolyzing activity (GSH) or is a variant that has been engineered to have GSH activity. In some embodiments, GSH activity is advantageous because the enzymes act to break down more of the starch in the molasses, particularly any granular (raw) starch. Alpha amylases having GSHE activity include, but are not limited to: those obtained from *Aspergillus kawachi* (e.g., AkAA), *Aspergillus niger* (e.g., AnAA), and *Trichoderma reesei* (e.g., TrAA). In some embodiments, the alpha amylase is an acid stable alpha amylase which, when added in an effective amount, has activity in the pH range of 3.0 to 7.0.

Further, in some embodiments, the alpha amylase can be a wild-type alpha amylase, a variant or fragment thereof or a hybrid alpha amylase which is derived from for example a catalytic domain from one microbial source and a starch binding domain from another microbial source. Non-limiting Examples of other alpha amylases that can be useful in combination with the blend are those derived from *Bacillus, Aspergillus, Trichoderma. Rhizopus. Fusarium, Penicillium. Neuraspora* and *Humicala.*

Some amylases usable as GSHE or secondary enzymes in the processes are commercially available, see, e.g., TERMAMYL® 120-L. LC and SC SAN SUPER®, SUPRA®, and LIQUEZYME® SC available from Novo Nordisk A/S, FUELZYME® LF from Verenium, and CLARASE® L, SPEZYME® FRED, SPEZYME® XTRA, GC626, and GZYME® G997 available from Danisco, US, Inc., Genencor Division.

It is not intended that the present invention be limited to any specific alpha amylase, as any suitable alpha amylase finds use in the methods of the present invention. Indeed, it is not intended that the present invention be limited to the specifically recited alpha amylase and commercial enzymes.

### Cellulases, hemicellulases and β-glucosidases

Various cellulases find use in the methods according to the invention. Cellulases are enzyme compositions that hydrolyze cellulose (β-1, 4-D-glucan linkages) and/or derivatives thereof, such as phosphoric acid swollen cellulose. Cellulases include the classification of exo-cellobiohydrolase (CBH), endoglucanases (EG) and β-glucosidases (BG) (see e.g., EC3.2.1.91, EC3.2.1.4 and EC3.2.1.21 for classification of these enzymes). Examples of cellulases include cellulases from *Penicillium, Trichoderma, Humicola, Fusarium, Thermomonaspora, Cellulomonas, Hypocrea, Clostridium, Thermonospore, Bacillus, Cellulomonas* and *Aspergillus.* Non-limiting examples of commercially available cellulases sold for feed applications are beta-glucanases such as ROVABIO® (Adisseo), NATUGRAIN® (BASF), MULTIFECT® BGL (Danisco US, Inc., Genencor Division) and ECONASE® (AB Enzymes). An exemplary commercial cellulase includes ACCELLERASE®, The cellulases and endoglucanases described in US20060193897AI also may be used. Beta-glucosidases (cellobiases) hydrolyze cellobiose into individual monosaccharides.

Hemicellulases are enzymes that break down hemicellulose. Hemicellulose categorizes a wide variety of polysaccharides that are more complex than sugars and less complex than cellulose, and are found in plant walls.

Numerous cellulases have been described in the scientific literature, examples of which include from *Trichoderma reesei:* Shoemaker, S. et al., Bio/Technology, 1:691-696, 1983, which discloses *CBHI;* Teeri, T. et al., Gene, 51:43-52, 1987, which discloses *CBHII;* Penttila, M. et al., Gene, 45:253-263, 1986, which discloses *EGI;* Salcheimo, M. et al., Gene, 63:11-22, 1988, which discloses *EGII;* Okada, M. et al., Appl. Environ. Microbiol., 64:555-563, 1988, which discloses *EGIII;* Saloheimo, M. et al., Eur. J. Biochem., 249:584-591, 1997, which discloses *EGIV;* Saloheimo, A. et al., Molecular Microbiology, 13:219-228, 1994, which discloses *EGV;* Barnett, C. C., et al., Bio/Technology, 9:562-567, 1991, which discloses *BGL1,* and Takashima, S. et al., J. Biochem., 125:728-736, 1999, which discloses *BGL2.* Cellulases from species other than Trichoderma have also been described e.g., Ooi *et al.,* 1990, which discloses the cDNA sequence coding for endoglucanase F1-CMC produced by *Aspergillus aculeatus;* Kawaguchi T *et al.,* 1996, which discloses the cloning and sequencing of the cDNA encoding beta-glucosidase 1 from *Aspergillus aculeatus;* Sakamoto *et al.,* 1995, which discloses the cDNA sequence encoding the endoglucanase CMCase-1 from *Aspergillus kawachii* IFO 4308; Saarilahti *et al.,* 1990 which discloses an endoglucanase from *Erwinia carotovara;* Spilliaert R, *et al.,* 1994, which discloses the cloning and sequencing of bg1A, coding for a thermostable beta-glucanase from *Rhodothermus marinu;* and Halldorsdottir S *et al.,* 1998, which discloses the cloning, sequencing and overexpression of a *Rhodothermus marinus* gene encoding a thermostable cellulase of glycosyl hydrolase family 12. It is not intended that the present invention be limited to any specific cellulase, as any suitable cellulase finds use in the methods of the present invention. Indeed, it is not intended that the present invention be limited to the specifically recited cellulases and commercial enzymes.

### β-glucanases

Various beta-glucanases find use in the invention in combination with transglucosidases. Beta-glucanases (endo-cellulase) also called endoglucanase I, II, and III, are enzymes that will attack the cellulose fiber to liberate smaller fragments of cellulose which is further attacked by exo-cellulase to liberate glucose. β-glucanases can also be used in the methods according to the invention. Commercial beta-glucanases useful in the methods of the invention include OPTIMASH® BG and OPTIMASH® TBG (Danisco, US, Inc. Genencor Division). It is not intended that the present invention be limited to any specific beta-glucanase, as any suitable beta-glucanase finds use in the methods of the present invention.

### Acid Fungal Proteases -

Various acid fungal proteases (AFP) find use in the methods of the invention. Acid fungal proteases include for example, those obtained from *Aspergillus, Trichoderma, Mucor* and *Rhizopus,* such as *A. niger, A. awamori, A. oryzae* and *M. miehei.* AFP can be derived from heterologous or endogenous protein expression of bacteria, plants and fungi sources. In particular, AFP secreted from strains of *Trichoderma* find use in the invention. Suitable AFP includes naturally occurring wild-type AFP as well as variant and genetically engineered mutant AFP. Some commercial AFP enzymes useful in the invention include FERMGEN® (Danisco US, Inc, Genencor Division), and FORMASE® 200.

In some embodiments, the acid fungal protease useful in the invention will have at least about 85%, 90%, 92%, 94%, 95%, 96%, 97%, 98% and 99% sequence identity to the amino acid sequence of SEQ ID NO:14 in United States Patent application 11/312,290, filed December 20, 2005. It is not intended that the present invention be limited to any specific acid fungal protease, as any suitable acid fungal protease finds use in the methods of the present invention. Indeed, it is not intended that the present invention be limited to the specifically recited acid fungal protease and commercial enzymes.

### Other Secondary Enzymes -

While some embodiments of the invention include a composition or blend of at least one transglucosidase, and optionally an alpha amylase, a glucoamylase, an AFP, a cellulase, a β-glucanase, and a pectinase, the blend or composition can optionally include other secondary enzymes. For example, when the blends are used in various applications (e.g. molasses processing applications) other secondary enzymes can be included. The blend or composition according to the invention can be used in a pretreatment step and/or the fermenting step along with the fermenting microorganism and other components and other secondary enzymes. The other secondary enzymes include without limitation: xylanases, other proteases (other than AFP), phytases, pectinases, pullulanases, beta amylases, lipases, cutinases, esterases, cyclodextrin transglycosyltransferases (CGTases), alpha galactosidases, dextrinases, beta-amylases and combinations thereof.

Any phytases are useful as secondary enzymes in the methods of the invention. Phytases are enzymes capable of liberating at least one inorganic phosphate from inositol hexaphosphate. Phytases are grouped according to their preference for a specific position of the phosphate ester group on the phytate molecule at which hydrolysis is initiated, (see e.g., 3-phytases (EC 3.1.3.8) or 6-phytases (EC 3.1.3.26)). A typical example of phytase is myo-inositol-hexakiphosphate-3-phosphohydrolase. Phytases can be obtained from microorganisms such as fungal and bacterial organisms (*e.g. Aspergillus (e.g., A. niger, A. terreus,* and *A. fumigalus*), *Myceliophthora (M. thermophila), Talaromyces (T. thermophilus) Trichoderma spp (T. reesei)* and *Thermomyces* (See e.g., WO 99/49740)). Also phytases are available from *Penicillium* species, (e.g., *P. hordei* (See e.g., ATCC No. 22053), *P. piceum* (See e.g., ATCC No. 10519), or *P. brevicompactum* (See e.g., ATCC No. 48944) (See, e.g. USP 6,475,762). Additional phytases that find use in the invention are obtainable from *Peniophora, E. coli, Citrobacter, Enterbacter* and *Buttiauxella* (see e.g., WO2006/043178, filed October 17, 2005). Additional phytases useful in the invention can be obtained commercially (e.g. NATUPHOS® (BASF), RONOZYME® P (Novozymes A/S), PHZYME® (Danisco A/S, Verenium) and FINASE® (AB Enzymes). In some embodiments, the phytase useful in the present invention is one derived from the bacterium *Buttiauxiella spp.,* such as BP-wt and variants (e.g. BP-17) (see United States Patent Application 12/027127, filed February 6, 2008). It is not intended that the present invention be limited to any specific phytase, as any suitable phytase finds use in the methods of the present invention.

In some embodiments, xylanases find use as secondary enzymes in the methods of the invention. Any suitable xylanase can be used in the invention. Xylanases (see, e.g. endo-β-xylanases (E.C. 3.2.1.8), which hydrolyze the xylan backbone chain, can be from bacterial sources (e.g., *Bacillus, Streptomyces, Clostridium, Acidothermus, Microtetrapsora or Thermonospora*) or from fungal sources *(Aspergillus, Trichoderma, Neurospora, Numicola, Penicillium* or *Fusarium* (See, e.g., EP473 545; USP 5,612,055; WO 92/06209; and WO 97/20920 for xylanases from some of these sources)). Xylanases useful in the invention include commercial blends (e.g., MULTIFECT® and FEEDTREAT® Y5 (Danisco US, Inc., Genencor Division), RONOZYME® WX (Novozymes A/S) and NATUGRAIN® WHEAT (BASF). In some embodiments the xylanase is from *Trichoderma reesei* or a variant xylanase from *Trichoderma reesei,* or the inherently thermostable xylanase described in EP1222256B1, as well as other xylanases from *Aspergillus niger, Aspergillus kawachii, Aspergillus tubigensis, Bacillus circulans, Bacillus pumilus, Bacillus subtilis, Neocallimastix patriciarum*, *Penicillium species, Streptomyces lividans, Streptomyces thermoviolaceus, Thermomonospora fusca, Trichoderma harzianum, Trichoderma reesei, Trichoderma viride.*

Additional proteases can also be used with the blends and/or compositions according to the invention other than AFPs. Any suitable protease can be used. Proteases can be derived from bacterial or fungal sources. Sources of bacterial proteases include proteases from *Bacillus* (e.g., *B. amyloliquefaciens, B. lentus, B. licheniformis,* and *B. subtilis*). Exemplary proteases include, but are not limited to, subtilisin such as a subtilisin obtainable from *B. amyloliquefaciens* and mutants thereof (USP 4,760,025). Suitable commercial protease includes MULTIFECT® P 3000 (Danisco US Inc., Genencor Division) and SUMIZYME® FP (Shin Nihon). Sources of suitable fungal proteases include, but are not limited to, *Trichoderma, Aspergillus, Humicola* and *Penicillium,* for example.

### Blends/Compositions -

The blends and compositions of the invention include at least one transglucosidase. In some embodiments, the transglucosidase is used in combination with at least one granular starch hydrolyzing enzyme (GHSE). In other embodiments, the granular starch hydrolyzing enzyme is a glucoamylase or an alpha amylase. In other embodiments, the blends or compositions of the invention include at least one transglucosidase, at least one alpha amylase with GSH activity, and at least one glucoamylase. In some embodiments both the glucoamylase and the alpha amylase have GSH activity. In other embodiments either the AA or GA has GSH activity. In some embodiments, the blends and/or compositions include a transglucosidase and a GSHE in combination with at least one other enzyme, such as at least one cellulase, at least one pectinase, at least one beta-glucanase, at least one beta glucosidase, and at least one acid fungal protease (AFP). In some embodiments, the blends and compositions comprise a transglucoridase, a glucoamylase, an alpha amytase, and an acid fungal protease. The enzyme components can be used as a blended formulation comprising two or three enzyme components mixed together or the enzyme components can be individually added during a process step to result in a composition encompassed by the invention. The composition of the invention is used during a step in starch conversion such that formulation is maintained. This may involve adding the separate components of the composition in a time-wise manner such that the formulation is maintained, for example adding the components simultaneously. A formulation is the blend or composition of the enzymes such that they are included in a certain percentage. Some formulations are provided below.

A composition comprising a glucoamylase and an alpha amylase, which is useful according to the invention is STARGEN™ 001, which is a blend of an acid *stable Aspergillus kawachi* alpha amylase and an *Aspergillus niger* glucoamylase (available commercially from Danisco US, Inc., Genencor Division). To this can be added a transglucosidase as disclosed herein.

In some embodiments, the enzyme blend or compositions will include;
a) a TG, an AkAA and a GA;
b) a TG. an AA with GSH activity and a GA having GSH activity;
c) a TG, an AkAA, a GA, and an AFP;
d) a TO, an AA, a GA having GSH activity, and a cellulose.
e) a TO, an AA having GSH activity, a GA having GSH activity, a cellulase, and a pectinase;
f) a TG, an AA having GSH activity, a GA having GSH activity, a hemicellulase, a beta-glucanase, a beta glucosidase, a pectinase and an AFP;
g) TG. an AA, a GA, a cellulase, and an AFP; L
h) GC626, ACCELLERASE®, OPTIDEX® L400, TG-L500, and FERMGEN®.

Some enzyme formulations are defined hereinbelow. However, in some embodiments, the enzyme formulation is at least about 5 %w/w transglucosidase, including at least about 10%, 15%, 20%, 25%, 30%, 35%, 40%, 45%, 50%, 55%, 60%, 65%, 70%, 75%, 80%, 85%, 90% and 95% w/w. In some embodiments, the blend or composition contains from about 1% to about 99 % w/w transglucosidase, preferably from about 5% to about 95%, preferably from about 10% to about 80% w/w transglucosidase. It is understood by the skilled artisan that the amount of transglucosidase could be varied depending upon the amount of raffinose and stachyose in the molasses. For example, if the molasses has a large amount of raffinose and/or stachyose, a higher percentage of transglucosidase can be included. Further, the amount of any enzyme in the formulation can be varied depending on the pretreatment and/or fermentation temperature. If the pretreatment and/or fermentation is at a temperature closer to the optimal temperature of the enzyme, less of the enzyme can be used. In some embodiments, the enzyme formulation has at least about 5% w/w alpha amylase (optionally GSHE), including about 10%, 15%, 20%, 25%, 30%, 35%, 40%, 45%, 50%, 55%, 60%, 65%, 70%, 75%, 80%, 85%, 90%, and 95% w/w. In some embodiments, the enzyme formulation has between about 5% and about 95% w/w alpha amylase, preferably between about 10% and about 50% w/w. In some embodiments, the formulation has at least about 5% glucoamylase (optionally GSHE), including at least about 10%, 15%, 20%, 25%, and 30% w/w. In some embodiments, the enzyme formulation has between about 5% and 95% glucoamylase, preferably between about 10% and about 50% w/w. In some embodiments, the formulation has about 0-20% w/w beta-glucanase. In some embodiments, the formulation has at least about 1% w/w beta-glucanase, including about 2%, 3%, 5%, 10%, 15%, and 20% w/w. In some embodiments, the formulation has about 0-15% w/w xylanase, in some embodiments at least about 5% xylanase, including about 10%, 15%, 20%, 25%, and 30% w/w. In some embodiments, the formulation has from about 0-20% w/w pectinase. In some embodiments, the formulation has at least about 5% w/w pectination, including about 10%, 15%, 20%, 25% w/w, and 30% w/w. In some embodiments, the formulation has from about 0-10% w/w lysozyme. In some embodiments, the enzyme blend or formulation will include:
1). TG (about 50% w/w) + fungal alpha amylase with GSHE activity (about 30% w/w) + GA (about 10% w/w) + acid fungal protease (about 10% w/w).
2). TG (about 40% w/w) + fungal alpha amylase with GSHE activity (about 20% w/w) + GA (20% w/w) + beta-glucanase (about 10% w/w) + beta-glucosidase (about 10% w/w).
3). TG (about 30% w/w) + fungal alpha amylase with GSHE activity (about 30% w/w) + GA (20% w/w) + pectinase (about 20% w/w).
4). TG (about 25% w/w) + fungal alpha amylase with GSHE activity (about 30% w/w) + GA (about 30% w/w) + xylanase (about 15% w/w).
5). TG (60% w/w) + fungal alpha amylase with GSHE activity (about 20% w/w) + GA (about 10% w/w) + lysozyme (about 10% w/w).

Other mixtures and formulations will be readily apparent from the disclosure herein.

### Molasses

Historically, the term molasses referred specifically to the final effluent obtained in the preparation of sucrose by repeated evaporation, crystallization and centrifugation of juices from sugarcane and from sugar beets. Several types of molasses are recognized and, in general, any liquid feed ingredient that contains in excess of 43% sugars is termed molasses (See, e.g., Curtin, L.V. "Molasses - General Considerations" Molasses in Animal Nutrition, 1983, pp. 2-11, National feed Ingredients Association, West Des Moines, Iowa). A variety of types of molasses are currently produced, including cane molasses, beet molasses, citrus molasses, hemicellulose extract, and starch molasses. Cane molasses is a by-product of the manufacture or refining of sucrose from sugarcane (sugarcane molasses). Beet molasses is a by-product of the manufacture of sucrose from sugar beets. Citrus molasses is the partially dehydrated juices obtained from the manufacture of dried citrus pulp. Hemicellulose extract is a by-product of the manufacture of pressed wood, and starch molasses is a by-product of dextrose manufacture from starch derived from corn or grain sorghums where the starch is hydrolyzed by enzymes and/or acid.

The type of molasses utilized in the methods of the invention is not critical. In some embodiments, the type of molasses used in the methods of the present invention can be any molasses that contains at least one of the non-fermentable sugars raffinose and/or stachyose. Any "Molasses" or thick syrup produced as a by-product of the processing of sugarcane or other plant products can be used, including, but not limited to: blackstrap molasses (from sugarcane), high test (cane) molasses, refiners cane or beet molasses (a by-product of refining raw brown sugar to produce white sugar), beet molasses (from sugar beets), and citrus molasses (juices extracted from dried citrus pulp), to name a few In some embodiments, the molasses used in the methods of the invention contains stachyose and/or raffinose in significant amounts. In some embodiments, significant amounts include at least about 0.1% ds, 0.5% ds, 1.0% ds, 2% ds, 3% ds, 4% ds, and 5% ds. In some embodiments, the molasses is produced as a by-product of sugar production from cane and/or beets.

### Methods of Use

In some embodiments, the molasses is contacted with the transglucosidase during the fermentation of the molasses. In some embodiments, the molasses is contacted with the transglucosidase during a pretreatment of the molasses and before fermentation of the molasses. In some embodiments, the transglucosidase is added both during a pretreatment and during fermentation of the molasses. In some embodiments, the transglucosidase is contacted with the molasses as part of an enzyme blend or composition. The molasses can be contacted with the transglucosidase and/or enzyme blend or composition of the invention in a single dose or a split dose as long as the formulation of enzymes is maintained. Some exemplary formulations are provided below. Thus, a split dose means that the total dose in the desired formulation is added in more than one portion, including two portions or three portions. In some embodiments, one portion of the total dose is added at the beginning and a second portion is added at a specified time in the process. In some embodiments, at least a portion of the dose is added as a pretreatment. In some embodiments, the transglucosidase and/or enzyme blend or composition of the invention, particularly the transglucosidase alone can be immobilized on a column or solid substrate.

The enzyme blend or composition can be added during one or both of the pretreatment and fermentation or during the combined pretreatment and fermentation. In either case, the enzyme blend or composition can be added at a temperature below the gelatinization temperature of the granular starch in the molasses. In some embodiments, the pretreatment is conducted at a temperature below the gelatinization temperature of the granular starch. In some embodiments, the pretreatment is conducted at the temperature optimum of the transglucosidase but below the gelatinization temperature of the granular starch. In some embodiments, the pretreatment is conducted at the temperature optimum of at least one of the enzymes in the enzyme blend and/or composition, but below the gelatinization temperature of the granular starch. This may allow for the use of a lower dose of enzyme than would be needed if the method were conducted at the same temperature as the fermentation. In some embodiments, the pretreatment is conducted at a temperature optimal for the enzyme but, below the gelatinization temperature of granular starch.

The initial starch gelatinization temperature ranges for a number of granular starches include: barley (52°C to 59°C), wheat (58°C to 64°C), rye (57°C to 70°C), corn (62°C to 72°C), high amylose corn (67°C to 80°C), rice (68°C to 77°C), sorghum (68°C to 77°C), potato (58°C to 68°C), tapioca (59°C to 69°C) and sweet potato (58°C to 72°C). (See, e.g., J.J.M. Swinkels pg 32 - 38 in STARCH CONVERSION TECHNOLOGY, Eds Van Beynum et al., (1985) Marcel Dekker Inc. New York and The Alcohol Textbook 3rd ED. A Reference for the Beverage, Fuel and Industrial Alcohol Industries, Eds Jacques et al., (1999) Nottingham University Press, UK).

Thus, the pretreatment and/or fermentation can be at a temperature below the starch gelatinization temperature of the granular starch. In some embodiments, this temperature is held between 45°C and 70°C; in other embodiments, the temperature is held between 50°C and 70°C; between 55°C and 70°C; between 60°C and 70°C, between 60°C and 65°C; between 55°C and 65°C and between 55°C and 68°C. In further embodiments, the temperature is at least 45°C, 48°C, 50°C, 53°C, 55°C, 58°C, 60°C, 63°C, 65°C and 68°C. In other embodiments, the temperature is not greater than 65°C, 68°C, 70°C, 73°C, 75°C and 80°C.

The pretreatment and/or fermentation can be conducted at a pH ranging from pH 3.5 to 7.0; also at a pH range of 3.5 to 6.5; also at a pH range of 4.0 to 6.0 and in some embodiments at a pH range of 4.5 to 5.5.

In some embodiments the pretreated molasses is subjected to fermentation with fermenting microorganisms. In some embodiments, the contacting step (pretreatment) and the fermenting step can be performed simultaneously in the same reaction vessel or sequentially. In general, fermentation processes are described in The Alcohol Textbook 3rd ED, A Reference for the Beverage, Fuel and Industrial Alcohol Industries, Eds Jacques et al., (1999) Nottingham University Press, UK.

During fermentation, the fermentable sugars (dextrins e.g. glucose) in the molasses are used in microbial fermentations under suitable fermentation conditions to obtain end products, such as alcohol (e.g., ethanol), organic acids (e.g., succinic acid, lactic acid), sugar alcohols (e.g., glycerol), ascorbic acid intermediates (e.g., gluconate, DKG, KLG ), and amino acids (e.g., lysine).

In some embodiments, the fermentable sugars are fermented with a yeast at temperatures in the range of 15 to 40°C, 20 to 38°C, and also 25 to 35°C; at a pH range of pH 3.0 to 6.5; also pH 3.0 to 6.0; pH 3.0 to 5.5, pH 3.5 to 5.0 and also pH 3.5 to 4.5 for a period of time of 5 hrs to 120 hours, preferably 12 to 120 and more preferably from 24 to 90 hours to produce an alcohol product, preferably ethanol.

Yeast cells are generally supplied in amounts of 10⁴ to 10¹², and preferably from 10⁷ to 10¹⁰ viable yeast count per ml of fermentation broth. The fermentation will include in addition to a fermenting microorganism (e.g. yeast) nutrients, optionally acid and enzymes. In some embodiments, in addition to the raw materials described above, fermentation media will contain supplements including but not limited to vitamins (e.g. biotin, folic acid, nicotinic acid, riboflavin), cofactors, and macro and micro-nutrients and salts (e.g. (NH4)₂SO₄; K₂HPO₄; NaCl; MgSO₄; H₃BO₃; ZnCl₂; and CaCl₂).

### Fermenting organisms

Examples of fermenting organisms are ethanologenic microorganisms or ethanol producing microorganisms such as ethanologenic bacteria which express alcohol dehydrogenase and pyruvate dehydrogenase and which can be obtained from *Zymomonas moblis* (See e.g. USP 5,000,000; USP 5,028,539, USP 5,424,202; USP 5,514,583 and USP 5,554,520). In additional embodiments, the ethanologenic microorganisms express xylose reductase and xylitol dehydrogenase, enzymes that convert xylose to xylulose. In further embodiments, xylose isomerase is used to convert xylose to xylulose. In some embodiments, a microorganism capable of fermenting both pentoses and hexoses to ethanol are utilized. For example, in some embodiments the microorganism can be a natural or non-genetically engineered microorganism or in other embodiments the microorganism can be a recombinant microorganism.

In some embodiments, the fermenting microorganisms include bacterial strains from *Bacillus, Lactobacillus, E. coli, Erwinia, Pantoea* (e.g., P. citrea), *Pseudomonas* and *Klebsiella* (e.g. *K. oxytoca*)*.* (See e.g. USP 5,028,539, USP 5,424,202 and WO 95/13362). The fermenting microorganism used in the fermenting step will depend on the end product to be produced.

In further embodiments, the ethanol-producing microorganism is a fungal microorganism, such as a yeast and specifically *Saccharomyces* such as strains of *S. cerevisiae* (USP 4,316,956). A variety of *S. cerevisiae* are commercially available and these include but are not limited to FALI® (Fleischmann's Yeast), SUPERSTART® (Alltech), FERMIOL® (DSM Specialties), RED STAR® (Lesaffre) and Angel Alcohol yeast (Angel Yeast Company, China).

### Recovery of alcohol and other end Products

One end product of the instant fermentation process is an alcohol product, (e.g. ethanol). The end product produced according to the process can be separated and/or purified from the fermentation media. Methods for separation and purification are known, for example by subjecting the media to extraction, distillation and column chromatography. In some embodiments, the end product is identified and/or purified directly by submitting the media to high-pressure liquid chromatography (HPLC) analysis.

In further embodiments, the mash can be separated by, for example, centrifugation into the liquid phase and solids phase and end products such as alcohol and solids recovered. The alcohol can be recovered by means such as distillation and molecular sieve dehydration or ultra filtration.

In some embodiments, use of an enzyme blend or composition according to the invention in a method of ethanol production will result in a yield of ethanol that is greater than 8%, 10%, 12%. 14%, 16%, 17%, 18%, 19%, 20%, 21%, and 22% by volume.

In further embodiments, the aqueous phase after evaporation of the ethanol can be wholly or partly added to the molasses to dilute or to prepare the molasses for fermentation.

In further embodiments, by use of appropriate fermenting microorganisms as known in the art, the fermentation end product can include without limitation ethanol, glycerol, 1,3-propanediol, gluconate, 2-keto-D-gluconate, 2,5-diketo-D-gluconate, 2-keto-L-gulonic acid, succinic acid, lactic acid, amino acids and derivatives thereof. More specifically when lactic acid is the desired end product, a *Lactobacillus* sp. (*L. casei*) can be used; when glycerol or 1,3-propanediol are the desired end products *E.coli* can be used; and when 2-keto-D-gluconate, 2,5-diketo-D-gluconate, and 2-keto-L-gulonic acid are the desired end products, *Pantoea citrea* can be used as the fermenting microorganism. The above enumerated list are only examples and one skilled in the art will be aware of a number of fermenting microorganisms that can be appropriately used to obtain a desired end product.

### Experimental

The present invention is described in further detail in the following examples which are not in any way intended to limit the scope of the invention as claimed. The attached Figures are meant to be considered as integral parts of the specification and description of the invention. The following examples are offered to illustrate, but not to limit the claimed invention.

In the disclosure and experimental section which follows, the following abbreviations apply: % w/w (weight percent); °C (degrees Centigrade); H₂O (water); dH₂O (deionized water); dIH₂O (deionized water, Milli-Q filtration); g or gm (grams); µg (micrograms); mg (milligrams); kg (kilograms); µl (microliters); mL and ml (milliliters); mm (millimeters); µm (micrometer); M (molar); mM (millimolar); µM (micromolar); U (units); MW (molecular weight); sec (seconds); min(s) (minute/minutes); hr(s) (hour/hours); DO (dissolved oxygen); W/V (weight to volume); W/W (weight to weight); V/V (volume to volume); Genencor (Danisco US Inc, Genencor Division, Palo Alto, CA); Ncm (Newton centimeter), ETOH (ethanol). eq (equivalents); N (Normal); ds or DS (dry solids content); MT (metric ton).

In the following examples the materials and methods used were:

HPLC method for fermentation broth analysis Using the Agilent 1100 HPLC, a Bio-rad Aminex HPX-87H or Rezex RoA- organic acid column was used. An ESTD method was used as follows: the mobile phase was 0.005 mol/L H₂SO₄. The sample was withdrawn and diluted 10 times, and filtered using a 0.45 µm filter membrane. The injection volume was 20µL, pump flow was 0.6 ml/min, column thermostat temperature was 60°C; RID, and optical unit temperature was 35°C.

HPLC method for molasses analysis - ESTD method The same HPLC device was used for fermentation analysis using the following conditions: the mobile phase was distilled water, the sample was diluted 30X with distilled water and filtered using a 0.45um filter membrane, the injection volume was 20ul, the pump flow was 0.4ml/min, the column thermostat temperature was 85°C; RID the optical unit temperature was 30°C and the analysis method was ESTD.

Starch Content Determination of Whole Grains Grains were mixed with MOPS buffer (50 mM, pH 7.0) plus calcium chloride (5 mM) and the pH adjusted with Acetic Acid Solution (2N) Sodium hydroxide (2N); Acetate Buffer (pH 4.2) was prepared as follows: 200 ml of 2N acetic acid to 500 ml of water. Using a standardized pH meter, add 2N sodium hydroxide to the mixture until the buffer is 4.2 +/- 0.05. SPEZYME^{®} FRED (Danisco US, Inc., Genencor Division, alpha-amylase from *Bacillus licheniformis*) and OPTIDEX^{®} L-400 (Danisco US, Inc., Genencor Division, glucoamylase from *Aspergillus niger*) are added and the starch content determined by HPLC.

Carbohydrate and Alcohol Analysis by High Pressure Liquid Chromatographic (HPLC): The composition of the reaction products of oligosaccharides was measured by HPLC (Beckman System Gold 32 Karat Fullerton, CA equipped with a HPLC column (Rezex 8 u8% H, Monosaccharides), maintained at 50°C fitted with a refractive index (RI) detector (ERC-7515A RI Detector, Anspec Company Inc.). Saccharides were separated based on molecular weight. A designation of DP1 is a monosaccharide, such as glucose; a designation of DP2 is a disaccharide, such as maltose; a designation of DP3 is a trisaccharide, such as maltotriose and the designation "DP4⁺" is an oligosaccharide having a degree of polymerization (DP) of 4 or greater.

Transglucosidase activity (TGU) is defined as the activity of enzyme required to produce one micromole of panose per minute under the conditions of the assay. The production of transglucosidase activity can be assayed as follows: The enzyme is brought up in 100 mM sodium acetate buffer, pH 4.5, containing 4 mM para-nitrophenyl-α-glucoside and 1 mg/ml BSA. After 30 min incubation at 30°C the reaction is terminated by the addition of an equal volume 1 M sodium carbonate and OD₄₀₅ was recorded.

Alpha amylase activity (AAU) can be determined by the rate of starch hydrolysis, as reflected in the rate of decrease of iodine-staining capacity measured spectrophotometrically. One AAU of bacterial alpha-amylase activity is the amount of enzyme required to hydrolyze 10 mg of starch per min under standardized conditions.

Alpha-amylase activity can also be determined as soluble starch unit (SSU) and is based on the degree of hydrolysis of soluble potato starch substrate (4% DS) by an aliquot of the enzyme sample at pH 4.5, 50°C. The reducing sugar content is measured using the DNS method as described in Miller, G. L. (1959) Anal. Chem. 31:426-428.

Glucoamylase Activity Units (GAU) is determined by using the PNPG assay to measure the activity of glucoamylase. GAU is defined as the amount of enzyme that will produce 1 g of reducing sugar calculated as glucose per hour from a soluble starch substrate at pH 4.2 and 60°C

Phytase Activity (FTU) was measured by the release of inorganic phosphate. The inorganic phosphate forms a yellow complex with acidic molybdate/vandate reagent and the yellow complex was measured at a wavelength of 415 nm in a spectrophometer and the released inorganic phosphate was quantified with a phosphate standard curve. One unit of phytase (FTU) is the amount of enzyme that releases 1 micromole of inorganic phosphate from phytate per minute under the reaction conditions given in the European Standard (CEN/TC 327,2005-TC327WI 003270XX).

Cellulase activity (ECU) was determined in endo-cellulase units (ECU) by measuring the ability of the enzyme to reduce the viscosity of a solution of carboxymethyl cellulose (CMC), The ECU assay quantifies the amount of catalytic activity present in the sample by measuring the ability of the sample to reduce the viscosity of a solution of carboxymethyl cellulose (CMC). The assay is carried out in a vibration viscometer (*e.g.,* MIVI 3000 from Sofraser, France) at 40°C; pH 7.5; 0.1 M phosphate buffer; Time = 30 min. using a relative enzyme standard for reducing the viscosity of the CHIC substrate (Hercules 7 LED), enzyme concentration approximately 0.15 ECU/mL). The arch standard is defined to 8200 ECU/g. One ECU is amount of enzyme that reduces the viscosity to one half under these conditions.

Protease_activity (SAPU) (spectrophotometric acid protease unit) was measure by the breakdown of casein wherein in 1 SAPU is the amount of protease enzyme activity that liberates one micromole of tyrosine per minute from a casein substrate under conditions of the assay.

Materials - molasses sugarcane molasses was obtained from Myanmar Great Golden Glory and Siam Chemicals, Thailand. Characterization of the molasses was determined and is summarized in Table 1:

**Table 1. Typical Composition of molasses:**

| **Composition** | | **Percent by weight ds** |
|---|---|---|
| Total Solids | | 80-90 |
| Total Reducing Sugars | | 45-52 |
| Total Proteins (% nitrogen X 6.25) | | 4-6 |
| Non-fermentable Sugar (starch, dextrans, cellulose, homicellulose, lignin, pectin) | | 4-5 |
| Non-fermentable | oligosaccharides; | 3-5 |
| Raffinose, | mg/gds | 0.4-1.0 |
| Stachyose, mg/gds | | 0.2-0.6 |
| Fermentable Sugars | | 41-47 |
| Total inorganics (Ash) | | 2-12 |
| pH | | 4.0-5.5 |
| Specific gravity | | 1.38-1.58 |

The starch content of the molasses used in the following experiments was determined to be 0.5%w/w.

Materials -enzymes - The following enzymes were used in the examples: TRANSGLUCOSIDASE L-500, GC626 (alpha amylase), GA-L (glucoamylase), MULTIFECT® (pectinase), GC220 (cellulase), ACCELLERASE® (hemicellulase), and FERMGEN® (AFP). All were obtained from Danisco US, Inc. Genencor Division.

In the following examples the effect of transglucosidase on molasses was tested to identify how the enzyme was increasing ethanol yield and to elucidate how much the ethanol yield was increased. Further tests were performed to identify secondary enzymes that could increase the efficiency of ethanol fermentation in the presence of transglucosidase.

### EXAMPLE 1

### Effect of transglucosidase on raffinose

In Example 1, the effect of transglucosidase (TRANSGLUCOSIDASE L-500 from Danisco US, Inc. Genencor Division) was tested on raffinose and stachyose.

Transglucosidases are generally known as transferases rather than as enzymes that hydrolyze sugars. More specifically, transglucosidases are known for converting maltooligosaccharides into isomalto-oligosaccharides such as isomaltose and panose which are less fermentable sugars. Thus, to test the theory that transglucosidase was able to hydrolyze non-fermentable oligosaccharides such as raffinose and stachyose into fermentable sugars, transglucosidase was tested directly on raffinose and stachyose as follows:

A solution of 1 % Raffinose and 1% Stachyose (from Sigma) was each separately incubated with TRANSGLUCOSIDASE L-500 at pH 4.5, 60°C and samples were withdrawn for HPLC analysis. The results in Figure 1 showed that glucose, sucrose, galactose and fructose (fermentable sugars) were produced from the hydrolysis of raffinose by the added TRANSGLUCOSIDASE L-500. The results for Stachyose were similar in that the sugars produced from the hydrolysis were the same (glucose; sucrose, galactose, and fructose), while the ratio or amounts of each differed. Thus, transglucosidases act to hydrolyze non-fermentable sugars such as raffinose and stachyose into fermentable sugars.

In Example 2, the effect of transglucosidase on sugarcane molasses fermentation was tested.

### EXAMPLE 2

### Effect of transglucosidase on molasses fermentation

Five hundred grams of sugarcane molasses (Myanmar Great Golden Glory and Siam Chemicals, Thailand) was diluted with 1000 grams tap water to a final DS of 25% and then adjusted to pH to 4.8 using 20% sulfuric acid (the molasses mixture). Yeast was prepared by using 1 gram active dry yeast (Dry Angel, Angel alcohol company) and mixing with 10 grams of tap water and 0.1-0.2% (w/w) sucrose, and holding at 30°C for 2 hrs to allow the yeast to propagate. 7 mls of the resulting yeast was added per 32.7 grams of the molasses mixture above with and without transglucosidase. Fermentations were conducted in a 150 ml Erlenmeyer flask in a 32°C bath with an agitation speed of 150 rpm. The fermentations were terminated at 42 hours by removing samples of the beer and conducting a distillation. Some of the samples were used for HPLC analysis. Distillation of the fermentation broth was carried out for calculating the ethanol yield per metric ton (MT) of molasses. TRANSGLUCOSIDASE L-500 (Danisco US, Inc, Genencor Division) was added at 6 ppm. The control contained no enzyme. The results are shown in Table 2 (molasses from Myanmar) and Table 3 (molasses from Thailand). In both Tables the ethanol yield is given with respect to IMT molasses to 95.5% ethanol (L) at 20°C. When using conventional methods to distill ethanol, 95.5% is the maximum amount that can be achieved at 20°C. The abbreviations used in the Table are as follows: TG (Transglucosidase); Gluc (Glucose); Fruc (Fructose); Suc acid (Succinic acid); Lac acid (Lactic acid); Glyc (Glycerol); Acet acid (Acetic acid); EtOH (ethanol).

**Table 2: Effect of transglucosidase on fermentation yield-molasses from Myanmar**

| TG | Time (h) | % w/v DP >3 | % w/v DP-3 | % w/v DP-2 | % w/v Gluc | % w/v Fruc | % w/v Suc acid | % w/v Lac acid | % w/v Glyc | % w/v acet acid | % v/v EtOH | EtOH yield/ MT |
|---|---|---|---|---|---|---|---|---|---|---|---|---|
| - | 41.0 | 2.23 | 0.05 | 0.08 | 0.41 | 0.69 | 0.25 | 0.37 | 1.47 | 0.25 | 9.88 | 258.8 |
| TG-L500 | 41.0 | 2.04 | 0.02 | 0.08 | 0.37 | 0.71 | 0.23 | 0.36 | 1.37 | 0.24 | 9.93 | 271.2 |

**Table 3: Effect of transglucosidase on fermentation yield-molasses from Thailand**

| TG | Time (h) | % w/v DP >3 | % w/v DP-3 | % w/v DP-2 | % w/v Gluc | % w/v Fruc | % w/v suc acid | % w/v lac acid | % w/v glyc | % w/v acet acid | % v/v EtOH | EtOH yield/ MT |
|---|---|---|---|---|---|---|---|---|---|---|---|---|
| - | 41.0 | 1.73 | 0.02 | 0.15 | 0.31 | 0.75 | 0.03 | 0.36 | 1.06 | 0.20 | 8.32 | 227.8 |
| TG-L500 | 41.0 | 1.60 | 0.18 | 0.14 | 0.32 | 0.79 | 0.06 | 0.35 | 1.26 | 0.22 | 8.61 | 233.0 |

The results in Table 2 and Table 3 confirmed the initial finding that the addition of TRANSGLUCOSIDASE® L-500 to molasses resulted in an increase in alcohol yield in molasses fermentations. Further, the increase in alcohol yield was seen irrespective of the origin of the molasses. Thus, the ability of the TRANSGLUCOSIDASE® L-500 to hydrolyze non-fermentable sugars in the molasses into glucose, sucrose, galactose and fructose (fermentable sugars), provided more fermentable sugars for the production of alcohol. This resulted in a higher alcohol yield.

### EXAMPLE 3

### The effect of raw starch hydrolyzing enzymes on molasses fermentations

The effect of the addition of raw starch hydrolyzing enzymes during fermentation of molasses by yeast was tested. Yeast fermentations were carried out as in Example 2 on molasses from Thailand. The raw starch hydrolyzing enzyme, GC 626 (alpha amylase from Danisco US, Inc., Genencor Division) was added to the yeast fermentation at 6 ppm. GC 626 is a granular starch hydrolyzing enzyme (GHSE). The fermentation broth at 41.5 hours was analyzed by HPLC and distilled for alcohol content (Table 4). In Table 4 the ethanol yield is given with respect to 1 MT molasses to 95.5% ethanol (L) at 20°C. The abbreviations used in the Table are as follows: TG (Transglucosidase); Gluc (Glucose); Fruc (Fructose); Suc acid (Succinic acid); Lac acid (Lactic acid); Glyc (Glycerol); Acet acid (Acetic acid); EtOH (ethanol).

**Table 4: Effect of raw starch hydrolyzing amylase during yeast fermentation containing molasses on the final alcohol yield**

| GSHE | Time (hr) | % w/v DP >3 | % w/v DP-3 | % w/v DP-2 | % w/v Gluc | % w/v Fruc | % w/v suc acid | % w/v lac acid | % w/v glyc | % w/v ace acid | % v/v EtOH | EtOH yield/ MT |
|---|---|---|---|---|---|---|---|---|---|---|---|---|
| - | 41.5 | 2.16 | 0.04 | 0.00 | 0.22 | 0.68 | 0.26 | 0.22 | 2.20 | 0.15 | 8.90 | 251.7 |
| GC626 | 41.5 | 1.96 | 0.03 | 0.00 | 0.24 | 0.64 | 0.23 | 0.35 | 1.32 | 0.36 | 9.30 | 264.1 |

The results in Table 4 show that the addition of the granular starch hydrolyzing enzyme (GSHE) to the molasses fermentation increased alcohol production. This is likely because hydrolysis of the granular starch in molasses resulted in increased fermentable sugars.

### EXAMPLE 4

### Formulation with GC626 and other secondary enzymes

Molasses from Thailand was used in the fermentations. The fermentations were conducted as in Example 2. Two formulations of secondary enzymes identified as formulations C and D in Table 5 were prepared. The amounts of the enzymes that were included in each formulation are set out in Table 5, however, the formulations typically included mixtures of the following enzymes: glucoamylase (GA-L® from Danisco US, Inc., Genencor Division); Pectinase (MULTIFECT® Pectinase from (Danisco US, Inc., Genencor Division); Cellulase (GC220 cellulose from Danisco US, Inc., Genencor Division); and Hemicellulase (ACCELLERASE® from Danisco US, Inc., Genencor Division). These enzymes were added to a molasses fermentation using the method in Example 2. The effect on fermentation of molasses was analyzed with and without 3 ppm of acid fungal protease. Since there is quite a lot of protein in molasses, it was hypothesized that protease might increase the efficiency of the fermentation by increasing the enzymes access to the starch molecules. Thus, formulation C+ in Table 6 included 3 ppm of FERMGEN® an acid fungal protease from Danisco US, Inc., Genencor Division in admixture with 3 ppm formulation C. In Table 6 the ethanol yield is given with respect to IMT molasses to 95.5% ethanol (L) at 20°C. The abbreviations used in the Table are as follows: TG (Transglucosidase); Gluc (glucose); Fruc (Fructose); Sue acid (Sucoinic acid); Lac acid (Lactic acid); Glyc (Glycerol); Acet acid (Acetic acid); EtOH (ethanol).

**Table 5: information on the enzyme formulation C and D**

| | GC626 (g) | GA-L-(g) | PECTINASE (g) | ACCELARASE (g) | Sum (g) |
|---|---|---|---|---|---|
| C | 30.352 | 5.0888 | 5.0157 | 5.98 | 46.4365 |
| D | 15.1495 | 7.6856 | 15.351 | 7.3383 | 45.5244 |

**Table 6: Fermentation results by HPLC and distillation:**

| blend | Time (h) | % w/v DP >3 | % w/v DP-3 | % w/v DP-2 | % w/v Gluc | % w/v Fruc | % w/v suc acid | % w/v lac acid | % w/v glyc | % w/v acet acid | % v/v EtOH | EtOH yield/ MT |
|---|---|---|---|---|---|---|---|---|---|---|---|---|
| - | 41.0 | 2.27 | 0.06 | 0.00 | 0.38 | 0.62 | 0.09 | 0.46 | 1.70 | 0.20 | 9.41 | 252.5 |
| C | 41.0 | 2.02 | 0.04 | 0.07 | 0.36 | 0.63 | 0.13 | 0.37 | 1.43 | 0.22 | 9.62 | 266.2 |
| D | 41.0 | 2.04 | 0.03 | 0.07 | 0.41 | 0.62 | 0.22 | 0.37 | 1.37 | 0.22 | 9.78 | 266.9 |
| C+ FERM GEN | 41.0 | 2.08 | 0.05 | 0.06 | 0.37 | 0.63 | 0.14 | 0.38 | 1.41 | 0.25 | 9.96 | 271.2 |

Table 6 shows the results of HPLC analysis and distillation. As seen in the table, each of the blends of enzymes (C, D and C+ FERMGEN) resulted in a more effective fermentation and increased fermentation efficiency. Without being held to a particular theory, the alpha-amylase (GC626) likely hydrolyzed raw starch residue in the molasses, while the glucoamylase hydrolyzed higher sugars and oligosaccharides, the acid fungal protease likely hydrolyzed the protein in molasses, generating free amino nitrogen, which helped yeast growth.

It was of interest to analyze the combined effect of the addition of transglucosidase with these enzymes in molasses fermentation. Thus, in Example 5, transglucosidase was tested with these secondary enzymes in molasses fermentations.

### EXAMPLE 5

### Formulation of TG with other enzymes

Molasses from Thailand was used in the fermentations. The fermentations were conducted as in Example 2. Blends were prepared containing mixtures of transglucosidase, granular starch hydrolyzing enzymes (GC626), glucoamylase (GA), pectinase, cellulases and hemicellulases to create formulation E and F as listed in Table 7 below. Further, acid fungal protease from Danisco US, Inc., Genencor Division, FERMGEN®, was also added to formulations E and F at 6 ppm. In Table 8, the ppm values given in the E and F columns are for the total enzyme in the formulation. For example, E + FERMGEN contained 3 ppm E and 3 ppm FERMGEN. In Table 9 the ethanol yield is given with respect to IMT molasses to 95.5% ethanol (L) at 20°C. The abbreviations used in the Table are as follows: T (h) (Time in hours); TG (Transglucosidase); Gluc (glucose); Fruc (Fructose); Sue acid (Succinic acid); Lac acid (Lactic acid); Glyc (Glycerol); Acet acid (Acetic acid); EtOH (ethanol).

**Table 7: TG formulation**

| | TG-L500(g) | GC626 (g) | GA(g) | Accelerase(g) | Pectinase(g) | Sum(g) |
|---|---|---|---|---|---|---|
| E | 5.0289 | 30.0386 | 5.1415 | 5.11 | 5.1931 | 50.5121 |
| F | 15.0848 | 15.4977 | 7.2614 | 8.0705 | 15.0686 | 60.983 |

**Table 8: Addition of acid fungal protease (FERMGEN)**

| Blends | FERMGEN | E | F |
|---|---|---|---|
| 1# Blank | / | / | / |
| 3# E | / | 6ppm | / |
| 4# E+FERMGEN | 3ppm | 3ppm | / |
| 5# F | / | / | 6ppm |
| 6# F+FERMGEN | 3ppm | / | 3ppm |

**Table 9: Fermentation results by HPLC and distillation:**

| Blends | T (h) | % w/v DP >3 | % w/v DP-3 | % w/v DP-2 | % w/v Gluc | % w/v Fruc | % w/v suc acid | % w/v lac acid | % w/v glyc | % acet acid | % v/v EtOH | EtOH yield/ MT |
|---|---|---|---|---|---|---|---|---|---|---|---|---|
| Control | 41.0 | 2.04 | 0.00 | 0.33 | 0.29 | 0.75 | 0.00 | 0.00 | 1.33 | 0.36 | 8.49 | 228.0 |
| 3# E | 41.0 | 1.97 | 0.00 | 0.45 | 0.28 | 0.76 | 0.00 | 0.00 | 1.31 | 0.35 | 8.58 | 239.5 |
| 4# E+ FERM GEN | 41.0 | 1.93 | 0.00 | 0.26 | 0.00 | 0.99 | 0.00 | 0.00 | 1.26 | 0.47 | 8.31 | 234.8 |
| 5# F | 41.0 | 1.94 | 0.00 | 0.43 | 0.28 | 0.76 | 0.00 | 0.00 | 1.21 | 0.47 | 8.56 | 240.3 |
| 6# F+ FERM GEN | 41.0 | 1.98 | 0.00 | 0.26 | 0.00 | 1.00 | 0.00 | 0.00 | 120 | 0.45 | 8.46 | 237.1 |

Table 9 shows the results from both HPLC and distillation using the formulations E and F in the presence or absence of FERMGEN. The results showed that there was an increase in the amount of ethanol produced during the fermentation for both formulations E and F. However, the additional protease did not further help to increase the alcohol content, this might be due to the dilution of the more effective enzymes in the formulations E and F.

Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. A method for increasing the fermentation yield of molasses in a fermentation medium, wherein the molasses contains at least one of the non-fermentable sugars raffinose and/or stachyose, the method comprising contacting the molasses with an enzyme composition comprising at least one fungal transglucosidase capable of hydrolyzing non-fermentable sugars such as raffinose and/or stachyose.

2. The method of claim 1, wherein the molasses is blackstrap molasses from sugarcane, high test cane molasses, refiners cane or beet molasses produced as a byproduct of refining raw brown sugar to produce white sugar, beet molasses from sugar beets, and citrus molasses juices extracted from dried citrus pulp.

3. The method of claim 1 or claim 2, further comprising the addition of one or more secondary enzymes to hydrolyze granular starch, proteins and/or residual starch in the molasses.

4. The method of any one of the preceding claims, wherein fermentable sugars from molasses in the fermentation reaction are converted to obtain end products, such as an alcohol, an organic acid or a specialty biochemical.

5. The method of claim 4, wherein the alcohol is ethanol and/or the organic acid is lactic acid or citric acid and/or the specialty biochemical is an amino acid.

6. The method of any one of the preceding claims, wherein the method increases the fermentable sugars obtained from the molasses.

7. The method of claim 6, further comprising fermenting the fermentable sugars to end products in the presence of fermenting microorganisms.

8. The method of claim 7, wherein the contacting and fermenting occur simultaneously or wherein the contacting is a pretreatment.

9. The method of claim 7, wherein the molasses comprises non-fermentable starch and the contacting, pretreatment and/or fermenting occurs at a temperature below the gelatinization temperature of the granular starch.

10. The method of claim 9, wherein the non-fermentable starch is granular starch.

11. The method of claim 9, wherein:
(a) the contacting and/or pretreatment temperature is below 52°C; and/or
(b) the fermentation temperature is from 15 to 40°C; and/or
(c) the fermentation, contacting and pretreatment temperature is from 15 to 40°C; and/or
(d) the contacting and/or pretreatment is conducted at a pH from 2.0 to 7.0; and/or
(e) the fermentation is conducted at a pH from 3.5 to 7.0.

12. The method of claim 6, further comprising contacting the molasses with at least one granular starch hydrolyzing enzyme (GSHE), such as an alpha amylase or a glucoamylase.

13. The method of claim 6, further comprising contacting the molasses with at least one other enzyme, such as a hemicellulase, a cellulase, a pectinase, a protease, β-glucosidase, a non-GSHE alpha amylase, and/or a non-GSHE glucoamylase.

14. The method of claim 13, wherein the protease is an acid fungal protease, such as an acid fungal protease is from an Aspergillus or Trichoderma species.

15. A method of producing ethanol from molasses which comprises having treated molasses in a fermentation medium according to the method of any one of the preceding claims, and the further step of fermenting the molasses in the presence of a fermenting organism to produce ethanol.

## Patentansprüche

1. Verfahren zur Steigerung der Fermentationsausbeute von Melasse in einem Fermentationsmedium, wobei die Melasse zumindest einen der nicht fermentierbaren Zucker Raffinose und/oder Stachyose enthält, wobei das Verfahren das Inkontaktbringen der Melasse mit einer Enzymzusammensetzung umfasst, die zumindest eine Pilz-Transglucosidase umfasst, die in der Lage ist, nicht fermentierbare Zucker wie Raffinose und/oder Stachyose zu hydrolysieren.

2. Verfahren nach Anspruch 1, wobei die Melasse Schwarze Melasse aus Zuckerrohr, High-Test-Rohrmelasse, Refiner-Rohr- oder Rübenmelasse ist, hergestellt als Nebenprodukt des Raffinierens braunen Rohzuckers zwecks Herstellung weißen Zuckers, Rübenmelasse aus Zuckerrüben und Zitrusmelassesäften, extrahiert aus getrockneter Zitruspulpe.

3. Verfahren nach Anspruch 1 oder Anspruch 2, das weiterhin das Zusetzen eines oder mehrerer sekundärer Enzyme umfasst, um granuläre Stärke, Proteine und/oder Reststärke in der Melasse zu hydrolysieren.

4. Verfahren nach einem der vorstehenden Ansprüche, wobei fermentierbare Zucker aus Melasse in der Fermentationsreaktion umgewandelt werden, um Endprodukte, wie einen Alkohol, eine organische Säure oder eine Spezialbiochemikalie, zu erhalten.

5. Verfahren nach Anspruch 4, wobei der Alkohol Ethanol ist und/oder die organische Säure Milchsäure oder Zitronensäure ist und/oder die Spezialbiochemikalie eine Aminosäure ist.

6. Verfahren nach einem der vorstehenden Ansprüche, wobei das Verfahren die fermentierbaren Zucker vermehrt, die aus der Melasse erhalten werden.

7. Verfahren nach Anspruch 6, das weiterhin Fermentieren der fermentierbaren Zucker zu Endprodukten in der Gegenwart fermentierender Mikroorganismen umfasst.

8. Verfahren nach Anspruch 7, wobei das Inkontaktbringen und Fermentieren gleichzeitig erfolgen oder wobei das Inkontaktbringen eine Vorbehandlung ist.

9. Verfahren nach Anspruch 7, wobei die Melasse nicht fermentierbare Stärke umfasst und das Inkontaktbringen, die Vorbehandlung und/oder das Fermentieren bei einer Temperatur unterhalb der Gelierungstemperatur der granulären Stärke erfolgt bzw. erfolgen.

10. Verfahren nach Anspruch 9, wobei die nicht fermentierbare Stärke granuläre Stärke ist.

11. Verfahren nach Anspruch 9, wobei:
(a) die Inkontaktbringungs- und/oder die Vorbehandlungstemperatur unter 52 °C liegt bzw. liegen; und/oder
(b) die Fermentationstemperatur 15 bis 40 °C beträgt; und/oder
(c) die Fermentations-, die Inkontaktbringungs- und die Vorbehandlungstemperatur 15 bis 40 °C betragen; und/oder
(d) das Inkontaktbringen und/oder die Vorbehandlung bei einem pH-Wert von 2,0 bis 7,0 durchgeführt wird bzw. werden; und/oder
(e) die Fermentation bei einem pH-Wert von 3,5 bis 7,0 durchgeführt wird.

12. Verfahren nach Anspruch 6, das weiterhin das Inkontaktbringen der Melasse mit zumindest einem granuläre Stärke hydrolysierenden Enzym (GSHE), wie einer alpha-Amylase oder einer Glucoamylase, umfasst.

13. Verfahren nach Anspruch 6, das weiterhin das Inkontaktbringen der Melasse mit zumindest einem anderen Enzym, wie einer Hemicellulase, einer Cellulase, einer Pektinase, einer Protease, einer β-Glucosidase, einer Nicht-GSHE-alpha-Amylase und/oder einer Nicht-GSHE-Glucoamylase, umfasst.

14. Verfahren nach Anspruch 13, wobei die Protease eine saure Pilzprotease, wie eine saure Pilzprotease aus einer Aspergillus- oder Trichoderma-Spezies, ist.

15. Verfahren zur Herstellung von Ethanol aus Melasse, das das Behandeln von Melasse in einem Fermentationsmedium gemäß dem Verfahren nach einem der vorstehenden Ansprüche und den weiteren Schritt des Fermentierens der Melasse in der Gegenwart eines fermentierenden Organismus umfasst, um Ethanol herzustellen.

## Revendications

1. Procédé pour augmenter le rendement de fermentation de mélasse dans un milieu de fermentation, dans lequel la mélasse contient au moins l'un des sucres non fermentables raffinose et/ou stachyose, le procédé comprenant la mise en contact de la mélasse avec une composition enzymatique comprenant au moins une transglucosidase fongique capable d'hydrolyser des sucres non fermentables tels que le raffinose et/ou le stachyose.

2. Procédé selon la revendication 1, dans lequel la mélasse est une mélasse noire de canne à sucre, une mélasse de canne "high test", une mélasse de canne ou de betterave de raffineur produite sous la forme d'un sous-produit du raffinage de sucre roux brut pour préparer du sucre blanc, une mélasse de betterave à partir de betteraves sucrières, et des jus de mélasse d'agrumes extraits à partir de pulpe d'agrumes séchée.

3. Procédé selon la revendication 1 ou 2, comprenant en outre l'addition d'une ou plusieurs enzymes secondaires pour hydrolyser un amidon granulaire, des protéines et/ou un amidon résiduel dans la mélasse.

4. Procédé selon l'une quelconque des revendications précédentes, dans lequel les sucres fermentables de mélasse sont convertis dans la réaction de fermentation pour obtenir des produits finis, tels qu'un alcool, un acide organique ou un produit biochimique de spécialité.

5. Procédé selon la revendication 4, dans lequel l'alcool est l'éthanol et/ou l'acide organique est l'acide lactique ou l'acide citrique et/ou le produit biochimique de spécialité est un acide aminé.

6. Procédé selon l'une quelconque des revendications précédentes, lequel procédé augmente les sucres fermentables obtenus à partir de la mélasse.

7. Procédé selon la revendication 6, comprenant en outre la fermentation des sucres fermentables en produits finis en présence de micro-organismes de fermentation.

8. Procédé selon la revendication 7, dans lequel la mise en contact et la fermentation se réalisent simultanément ou dans lequel la mise en contact est un prétraitement.

9. Procédé selon la revendication 7, dans lequel la mélasse comprend un amidon non fermentable et la mise en contact, le prétraitement et/ou la fermentation se réalisent à une température en dessous de la température de gélatinisation de l'amidon granulaire.

10. Procédé selon la revendication 9, dans lequel l'amidon non fermentable est un amidon granulaire.

11. Procédé selon la revendication 9, dans lequel:
(a) la température de mise en contact et/ou de prétraitement est en dessous de 52°C; et/ou
(b) la température de fermentation est de 15 à 40°C; et/ou
(c) la température de fermentation, de mise en contact et de prétraitement est de 15 à 40°C; et/ou
(d) la mise en contact et/ou le prétraitement sont réalisés à un pH de 2,0 à 7,0; et/ou
(e) la fermentation est effectuée à un pH de 3,5 à 7,0.

12. Procédé selon la revendication 6, comprenant en outre la mise en contact de la mélasse avec au moins une enzyme hydrolysant un amidon granulaire (GSHE), telle qu'une alpha amylase ou une glucoamylase.

13. Procédé selon la revendication 6, comprenant en outre la mise en contact de la mélasse avec au moins une autre enzyme, telle qu'une hémicellulase, une cellulase, une pectinase, une protéase, une β-glucosidase, une alpha amylase non GSHE et/ou une glucoamylase non GSHE.

14. Procédé selon la revendication 13, dans lequel la protéase est une protéase fongique acide, telle qu'une protéase fongique acide provenant d'une espèce Aspergillus ou Trichoderma.

15. Procédé de production d'éthanol à partir de mélasse, qui comprend le traitement de mélasse dans un milieu de fermentation selon le procédé selon l'une quelconque des revendications précédentes, et l'étape supplémentaire de fermentation de la mélasse en présence d'un organisme de fermentation pour produire de l'éthanol.
